# EUROPEAN PATENT APPLICATION

(11) **EP 4 428 649 A2**
(43) Date of publication of application: **11.09.2024**
(21) Application number: 22914687.3
(22) Date of filing: 26.12.2022
(51) Int. Cl.: G05D 23/20, A61M 16/10

(54) **HEATING CONTROL CIRCUIT AND BREATHING MACHINE**

(30) Priority: 31.12.2021 CN 202123441555 U; 31.12.2021 CN 202111674664
(71) Applicant: BMC (Tianjin) Medical Co., Ltd., Tianjin 301700 (CN)
(72) Inventor: YANG, Wenyan, Beijing 100041 (CN); TI, Yao, Tianjin 301700 (CN); ZHENG, Fang, Tianjin 301700 (CN); TIAN, Xin, Tianjin 301700 (CN); WANG, Qingsong, Beijing 100041 (CN); ZHUANG, Zhi, Beijing 100041 (CN)
(74) Representative: Grünecker Patent- und Rechtsanwälte PartG mbB
(86) International application number: PCT/CN2022/142002
(87) International publication number: WO 2023/125433

(57) **Abstract**

The embodiments of the present invention belong to the technical field of electronic circuits. Provided are a heating control circuit and a breathing machine. The heating control circuit comprises a control device and a switch device, wherein an input end of the control device is connected to an alternating-current power source, an output end thereof is connected to a control end of the switch device, and an output end of the switch device is connected to a heating member; and the control device is configured to generate, according to different output voltages of the alternating-current power source, a driving signal for controlling the adaptive change of a duty ratio of the switch device, the adaptive change of the duty ratio enabling the power, which is output by the switch device to the heating member, to be kept constant. The heating control circuit in the present invention is directly connected to an alternating-current power source having different alternating-current voltages, thereby realizing the constant-power output of a heating member.

## Description

### CROSS REFERENCE TO RELATED APPLICATIONS

This application claims priority to Chinese patent applications No. 202123441555.4 and No. 202111674664.2 submitted on December 31, 2021, the contents of which are incorporated herein by reference.

### TECHNICAL FIELD

The present disclosure relates to the field of electronic circuit technology, in particular to a heating control circuit and a ventilator.

### BACKGROUND

In existing technology, the heating plate needs to be heated at a constant power. However, heating plates are sold to different countries where the external alternating current (AC) voltage connected to the heating plate varies. For instance, a grid power supply of 110V is adopted in some countries, while a grid power supply of 220V is adopted in other countries. The existing heating circuit for the heating plate achieves a constant power by directly detecting the zero-crossing point or duty ratio of the grid power supply based on a known voltage. However, when the voltage of the external grid power supply changes, the constant power calculated by such heating circuit is generally inaccurate, which can cause damage to the heating circuit and the heating plate.

### SUMMARY

The object of embodiments of the present disclosure is to provide a heating control circuit and a ventilator that can at least solve the technical problems mentioned above.

To achieve the above object, in a first aspect, an embodiment of the disclosure provides a heating control circuit, including: a control device and a switching device; an input end of the control device is connected to an AC power supply, and an output end of the control device is connected to a control end of the switching device; an output end of the switching device is connected to a heating member. The control device is configured to generate, according to different output voltages of the AC power supply, a driving signal for controlling a duty ratio of the switching device to adaptively change, so that the power output to the heating member remains constant.

Optionally, the switching device is a MOSFET. The gate of the MOSFET is connected to the control device, the source of the MOSFET is connected to the power ground terminal, and the drain of the MOSFET is connected to the input end of the heating member, among them, the gate of the MOSFET is configured as a control end.

Optionally, the heating control circuit further includes a voltage divider circuit connected in parallel with a series circuit formed by the heating member and the switching device, and the voltage divider circuit includes a first resistor and a second resistor connected in series.

Optionally, the heating control circuit further includes an isolation device disposed between the output end of the control device and the control end of the switching device.

Optionally, the heating control circuit further includes a third resistor disposed between the isolation device and the switching device.

Optionally, the heating control circuit further includes a rectifying device that is connected between the AC power supply and the heating member, and configured for converting an output voltage signal of the AC power supply into a DC voltage and providing the DC voltage to the heating member.

Optionally, the heating control circuit further includes a voltage acquisition device, that is connected between the AC power supply and the control device and is configured to sample the output voltage signal of the AC power supply to provide the same to the control device.

Optionally, the voltage acquisition device is a mutual inductor, the input end of the mutual inductor is connected to the AC power supply, and the output end is connected to the input end of the control device.

Optionally, the control device is a power calculation chip.

In a second aspect, an embodiment of the disclosure provides a ventilator including a heating member and any of the heating control circuits according to the first aspect.

A third aspect provides a method for maintaining power stability, including: determining a target power value and an actual maximum power value, the target power value representing a power value required to be stably output from a heating plate of a ventilation treatment equipment; outputting an overall control signal according to the target power value and the actual maximum power value. The overall control signal includes an enable signal and a disable signal. Each time the enable signal is output, the heating plate of the ventilation treatment equipment continuously operates for a preset duration, and each time the disable signal is output, the heating plate stops operating for a preset duration.

Optionally, the outputting an overall control signal according to the target power value and the actual maximum power value includes: determining a duty ratio based on the target power value and the actual maximum power value; and outputting the overall control signal according to the duty ratio.

Optionally, the outputting the overall control signal according to the duty ratio includes: determining the number of times that the overall control signal is output according to the duty ratio; determining, based on the duty ratio and the number of times that the overall control signal is output, the number of times that the enable signal is output and the number of times that the disable signal is output; outputting the enable signal according to the number of times that the enable signal is output, and outputting the disable signal according to the number of times that the disable signal is output.

Optionally, the outputting an overall control signal according to the target power value and the actual maximum power value includes: taking the target power value as an incremental value; outputting the overall control signal according to the incremental value and the actual maximum power value.

Optionally, the outputting the overall control signal according to the incremental value and the actual maximum power value includes: starting from 0, accumulating the incremental value once in each preset cycle, and comparing the accumulated value obtained from accumulating the incremental value once with the actual maximum power value in each preset cycle; in response to the accumulated value in any preset cycle being less than the actual maximum power value, outputting the disable signal in that preset cycle, wherein each time the disable signal is output, the heating plate stops operating for a predetermined duration; in response to the accumulated value in any preset cycle being not less than the actual maximum power value, outputting the enable signal in that preset cycle; in a preset cycle following the cycle during which the enable signal is output, performing the following steps based on a difference: accumulating the incremental value once in each preset cycle, and comparing the accumulated value obtained from accumulating the incremental value once with the actual maximum power value in each preset cycle, wherein the difference is a difference value of the actual maximum power value and the accumulated value in the preset cycle during which the enable signal is output.

Optionally, the determining the actual maximum power value includes: acquiring a working power supply value of the ventilation treatment equipment; determining the actual maximum power value according to the working power supply value and the resistance value of the heating plate.

Optionally, the resistance value is determined based on the following criteria: considering a standard voltage range of international civil electricity, ensuring the ventilation treatment equipment to continuously output the target power value while operating, and ensuring the actual maximum power value to be not less than the target power value.

Optionally, the method further includes: receiving a temperature signal from a temperature sensor, wherein the temperature signal represents a real-time temperature of the heating plate; in response to the real-time temperature being less than a preset temperature, executing steps of: outputting the overall control signal according to the target power value and the actual maximum power value; in response to the real-time temperature being not less than the preset temperature, continuously outputting the disable signal, and outputting the overall control signal according to the target power value and the actual maximum power value until the real-time temperature drops to less than the preset temperature.

A fourth aspect provides a device for maintaining power stability, including: a determination module, configured to determine a target power value and an actual maximum power value, the target power value representing a power value required to be stably output by ventilation treatment equipment; and an output module, configured to output an overall control signal based on the target power value and the actual maximum power value. The overall control signal includes an enable signal and a disable signal. Each time the enable signal is output, the heating plate of the ventilation treatment equipment continuously operates for a preset duration, and each time the disable signal is output, the heating plate stops operating for a preset duration.

Optionally, the output module includes: a duty ratio determination sub-unit, configured to determine the duty ratio based on the target power value and the actual maximum power value; and a first overall control signal output sub-unit, configured to output the overall control signal according to the duty ratio.

Optionally, the first overall control signal output sub-unit is further configured to: determine, based on the duty ratio, the number of times that the overall control signal is output; determine, based on the duty ratio and the number of times that the overall control signal is output, the number of times that the enable signal is output and the number of times that the disable signal is output; output the enable signal according to the number of times that the enable signal is output, and output the disable signal according to the number of times that the disable signal is output.

Optionally, the output module includes: an increment sub-unit, configured to take the target power value as an increment value; and a second overall control signal output sub-unit, configured to output the overall control signal based on the increment value and the actual maximum power value.

Optionally, the second overall control signal output sub-unit is further configured to: accumulate the increment value once in each preset cycle starting from 0, and compare the accumulated value obtained through accumulating the increment value once with the actual maximum power value in each preset cycle; in response to the accumulated value of any preset cycle being less than the actual maximum power value, output a disable signal in that preset cycle, wherein the heating plate stops operating for a preset duration each time the disable signal is output; in response to the accumulated value in any preset cycle being not less than the actual maximum power value, output an enable signal in that preset cycle; in a preset cycle following the cycle during which the enable signal is output, the following steps are performed based on a difference value: accumulating the increment value once in each preset cycle, and comparing the accumulated value obtained by accumulating the increment value once with the actual maximum power value in each preset cycle, wherein the difference value is a difference value of the actual maximum power value and the accumulated value in the preset cycle during which the enable signal is output.

Optionally, the determination module includes: an acquisition sub-unit, configured to acquire a working power supply value of the ventilation treatment equipment; and a determination sub-unit, configured to determine the actual maximum power value based on the working power supply value and the resistance value of the heating plate

Optionally, the device further includes: a temperature reception module, configured to receive a temperature signal from a temperature sensor, the temperature signal representing the real-time temperature of the heating plate; a first execution module, configured to execute the steps of outputting the overall control signal based on the target power value and the actual maximum power value when the real-time temperature is less than the preset temperature; and a second execution module, configured to continuously output the disable signal when the real-time temperature is not less than the preset temperature, and execute the step of outputting the overall control signal based on the target power value and the actual maximum power value until the real-time temperature drops below the preset temperature.

A fifth aspect provides a ventilation treatment equipment, including: a main control board; the main control board is configured to execute any method for maintaining power stability as described in the third aspect.

Through the above technical solutions, the heating control circuit of the present disclosure can be externally connected to an AC power supply with different AC voltages to achieve constant power output of the heating member, and its structure is simple.

According to the method for maintaining power stability provided by the present disclosure, the target power value and the actual maximum power value are determined first, and then an overall control signal is output based on the target power value and the actual maximum power value. The overall control signal includes an enable signal and a disable signal. Each time the enable signal is output, the heating plate continues to work for a predetermined duration, and each time the disable signal is output, the heating plate stops operating for a predetermined duration, thereby achieving the object of maintaining a stable power output of the heating plate.

In the present disclosure, based on the resistance value of the same heating plate, it is not necessary to consider the standard voltage range of civil AC voltage, and two power values are used to control the output of an enable signal and a disable signal, thereby achieving the object of stable power output of the heating plate. Since it is not necessary to design and manufacture heating plates with different resistance values, the cost of the ventilation treatment equipment is indirectly reduced. Moreover, when the voltage of the operating power supply fluctuates, the output power of the heating plate is not affected. In this way, based on the same resistance value of the heating plate, within the global standard voltage range of 100V to 240V, and even when the voltage of the operating power supply fluctuates, the object of maintaining stable power output of the heating plate is achieved, which has a high practical value.

The other features and advantages of the embodiments of the disclosure will be explained in detail in the section of "DETAILED DESCRIPTION OF THE EMBODIMENTS" below.

### BRIEF DESCRIPTION OF THE DRAWINGS

The accompanying drawings, which constitute a part of the specification, are used to provide a further understanding of the embodiments of the present disclosure, and explain the embodiments of the present disclosure together with the following specific embodiments, without limiting the embodiments of the present disclosure. In the accompanying drawings:
FIG.1 is a schematic block diagram of a heating control circuit according to an exemplary embodiment;
FIG. 2 is a schematic connection diagram of a heating control circuit according to an exemplary embodiment;
FIG. 3 is a schematic diagram illustrating a change of a 110V AC voltage signal according to an exemplary embodiment;
FIG. 4 is a schematic diagram illustrating a change of a 220V AC voltage signal according to an exemplary embodiment;
FIG. 5 is a flow chart of a method for maintaining power stability according to an embodiment of the disclosure;
FIG. 6 is a schematic diagram illustrating outputting an enable signal when method (2) for outputting the enable signal using an accumulation method is used in an embodiment of the disclosure;
FIG.7 is a schematic structural diagram of a ventilation treatment equipment according to an embodiment of the disclosure; and
FIG. 8 is a block diagram of a device for maintaining power stability according to an embodiment of the disclosure.

### DETAILED DESCRIPTION OF THE EMBODIMENTS

The specific implementation of the embodiments of the present disclosure is described in detail below in conjunction with the accompanying drawings. It should be understood that the specific implementation described here is only used to illustrate and explain the embodiments of the present disclosure, and is not used to limit the embodiments of the present disclosure.

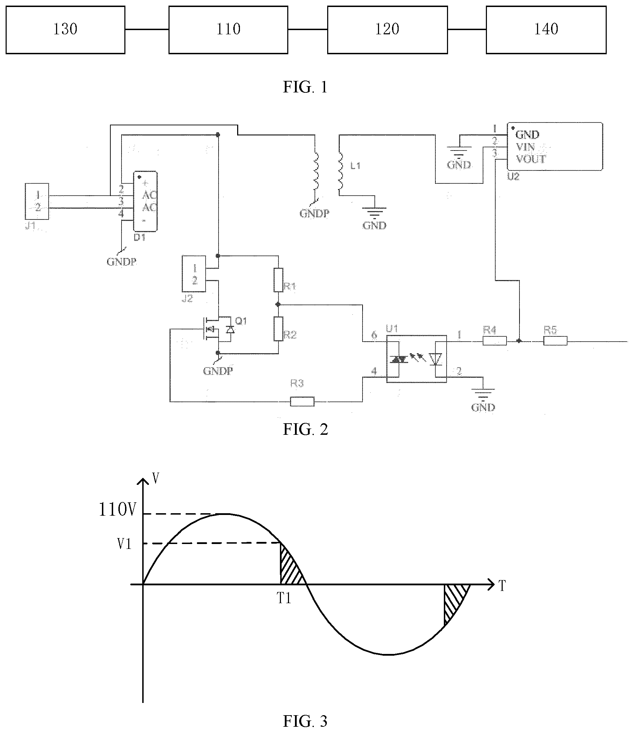

FIG.1 is a schematic block diagram of a heating control circuit according to an exemplary embodiment. As shown in FIG. 1, the heating control circuit includes a control device 110 and a switching device 120. The control device 110 has an input end connected to an AC power supply 130, and an output end connected to a control end of the switching device 120, and an output end of the switching device 120 is connected to a heating member 140. Among them, the control device 110 is configured to generate, according to different output voltages of the AC power supply 130, a driving signal for controlling a duty ratio of the switching device 120 to change adaptively, so that the power output from the switching device 120 to the heating member 140 remains constant.

The heating control circuit provided by the embodiment of the present disclosure can achieve constant power heating of the heating member by being externally connected to an AC power supply with different AC voltages, and has a simple structure and low cost.

In a preferred embodiment, the switching device 120 is a MOSFET (Metal Oxide Semiconductor Field Effect Transistor). The gate of the MOSFET is connected to the control device 110, the source is connected to the power ground terminal, and the drain is connected to the input end of the heating member 140, among them, the gate of the MOSFET is configured as a control end.

For example, the MOSFET may operate according to the basic operating principle of forward conduction and reverse cutoff, and may be turned on and turned off adaptively based on the driving signal of the control device 110. Specifically, when the MOSFET is turned on, the heating member 140 connected thereto starts heating, and when the MOSFET is turned off, the heating member 140 connected thereto stops heating. Accordingly, the MOSFET is controlled to be turned on or turned off regularly in a certain period, so that the heating member 140 remains a constant power output in a certain period.

The switching device 120 is a MOSFET. The gate of the MOSFET is connected to the control device 110, the source is connected to the power ground terminal, and the drain is connected to the input end of the heating member 140, among them, the gate of the MOSFET is configured as a control end.

Different types of MOSFETs such as a N-channel MOSFET and a P-channel N MOSFET may be selected according to actual needs in the embodiments of the present disclosure, which is not excessively limited here.

In a preferred embodiment, the heating control circuit further includes a voltage divider circuit connected in parallel with a series circuit formed by the heating member 140 and the switching device 110, and the voltage divider circuit includes a first resistor and a second resistor connected in series.

The voltage divider circuit formed by the first resistor and the second resistor connected in series can effectively control the voltage across the shunt, preventing excessive voltage from breaking down the MOSFET, and avoiding damage to the heating member. Moreover, in the embodiment of the present disclosure, the resistance values of the first resistor and the second resistor may be set according to the actual application of the circuit, and the types of the first resistor and the second resistor are not limited. For example, the first resistor and the second resistor may adopt sliding rheostats to control and adjust the voltage across the shunt by changing the size of the resistance.

In a preferred embodiment, the heating control circuit further includes an isolation device disposed between the output end of the control device 110 and the control end of the switching device 120.

Specifically, the isolation device is configured to effectively isolate the input and the output to prevent interference signals from affecting the circuit, and has good electrical insulation capabilities. For example, the isolation device may be a photo-coupler, for example a photo-coupler of the model MOC3063.

In a preferred embodiment, the heating control circuit further includes a third resistor disposed between the isolation device and the switching device. Specifically, the third resistor can limit the current of the signal output from the isolation device, protecting the switching device effectively and preventing damage to the switching device caused by excessive current.

In a preferred embodiment, the heating control circuit further includes a rectifying device that is connected between the AC power supply and the heating member, and configured for converting an output voltage signal of the AC power supply into a DC voltage and providing the DC voltage to the heating member.

In the embodiment of the present disclosure, the AC voltage is converted into the DC voltage through a rectifying device, which can meet the needs of the heating member for the DC voltage. More preferably, a filtering element may be further disposed between the voltage conversion device and the heating member in the embodiment of the present disclosure to filter the converted DC voltage signal, thereby effectively removing the interference signals in the DC voltage signal, and ensuring the safe use of the heating member.

Among them, the rectifying device may adopt a full-wave rectifier bridge, a half-wave rectifier bridge or other types of rectifier circuits according to actual application needs, and no excessive restrictions are made here.

In a preferred embodiment, the heating control circuit further includes a voltage acquisition device, that is connected between the AC power supply 130 and the control device 110 and is configured to sample the output voltage signal of the AC power supply 130 to provide it to the control device 110.

In a preferred embodiment, the voltage acquisition device is a mutual inductor having an input end connected to the AC power supply and an output end connected to the input end of the control device, which can reduce the AC voltage according to a certain ratio to meet the use of the control device.

In a preferred embodiment, the control device is a power calculation chip. The power calculation chip can be built-in with different power calculation formulas according to actual application needs to meet the user's various needs for constant power output. For example, the model of the power calculation chip includes HLW8012 and the like.

As can be seen from the above embodiments, the heating control circuit of the present disclosure may be connected to an AC power supply with various AC voltages. Thus, on the basis of achieving a constant power, the heating control circuit further meets the demand for a wide voltage, so that it can adapt to different application scenarios.

A process for achieving a constant power output of the heating control circuit is explained below in detail by taking a more specific embodiment as an example.

FIG. 2 is a schematic connection diagram of a heating control circuit according to an exemplary embodiment. The connection between various units and devices in FIG. 2 is briefly described first. As shown in FIG. 2, the rectifier bridge D1 includes alternating current voltage input terminals AC for connecting to an alternating current (AC) power supply J1. The positive output terminal 1 of the rectifier bridge D1 is connected to the input terminal 1 of the heating member. One input terminal of the mutual inductor L1 is connected to the AC power supply J1, and the other input terminal of the mutual inductor is connected to a power ground terminal GNDP. One output terminal of the mutual inductor is connected to the input terminal VIN of the power calculation chip U2, and the other output terminal of the mutual inductor is connected to a common ground terminal. The input terminal 1 of the photo-coupler U1 is connected to the output terminal VOUT of the power calculation chip U2, the output terminal 4 of the photo-coupler U1 is connected to the gate of the MOSFET via the third resistor R3, and the output terminal 6 of the photo-coupler U1 is connected between the first resistor R1 and the second resistor R2 in the voltage divider circuit. One end of the first resistor R1 is connected to the input terminal 1 of the heating member, and one end of the second resistor R2 is connected to the source of the MOSFET and connected to the power ground terminal GNDP. The drain of the MOSFET is connected to the input terminal 2 of the heating member.

The operating process of the heating control circuit is as follows:

Firstly, when the heating control circuit is connected to an AC power supply, the rectifier bridge converts an AC voltage signal of the AC power supply into a DC voltage and outputs the DC voltage to the heating member. The mutual inductor L1 collects the AC voltage of the AC power supply and transmits the collected voltage to the power calculation chip so that the power calculation chip calculates the power according to the collected voltage.

Secondly, the power calculation formula is built into the power calculation chip according to the constant power output requirement of the heating member in the disclosure. An integral operation method is specifically used to calculate the output power of the AC voltage. In addition, a power determination condition is also built into the power calculation chip for determining whether the output power exceeds a preset power threshold. The preset power threshold is the preset adaptive power of the heating member mentioned above.

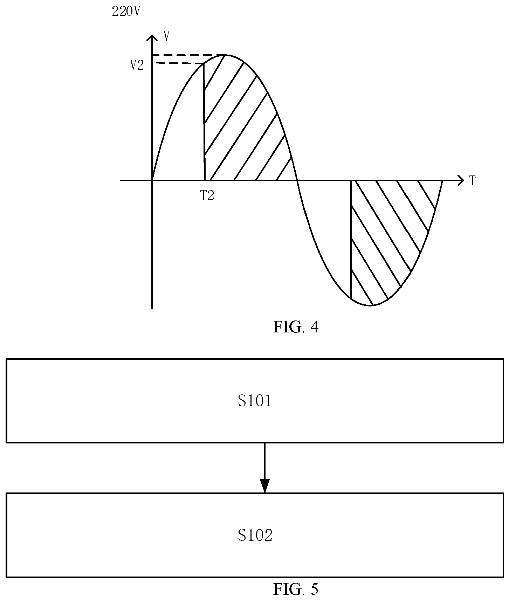

Specifically, referring to FIG. 3 and FIG. 4, FIG. 3 and FIG. 4 are schematic diagrams that respectively illustrate an AC voltage of 110V and an AC voltage of 220V. Description is made by taking an example with an AC voltage of 110V and a power threshold of 200W, when the power calculation chip detects the zero crossing point of the AC voltage, the power calculation chip starts to calculate the output power of the AC voltage. The AC voltage rises over time, and the power calculation chip performs an integral operation based on the power corresponding to the voltage value collected at a certain moment to obtain the output power at that moment. As shown in FIG. 3, the output power obtained by integrating the voltage V1 corresponding to the moment T1 is 200W. In FIG. 4, compared with the AC voltage of 110V, the voltage rise rate of the AC voltage of 220V at the same time is faster than that of the AC voltage of 110V. Accordingly, in FIG. 4, the output power obtained by integrating the voltage V2 corresponding to the moment T2 is 200W, and T2<T1. As can be known from the power calculation formula W=U²/R, if a 100% output is adopted for the AC voltage of 110V, then only 25% output is adopted for the AC voltage of 220V to achieve the same power output as that of 110V. Taking FIG. 3 and FIG. 4 as examples, the area of the white region in FIG. 3 is the same as the area of the white region in FIG. 4, and the area of the white region in FIG. 4 occupies 1/4 of the area of the semicircle formed by the half cycle of the AC power supply of 220V.

Furthermore, when the power calculation chip determines that the output power does not exceed the power threshold, the output driving signal is an enable signal. The photo-coupler outputs a corresponding output signal according to the enable signal, so that the MOSFET is turned on. At this time, the heating control circuit and the heating member form a closed loop, and the heating member can implement heating. When the power calculation chip determines that the output power exceeds the power threshold, the driving signal generated is a disable signal. Based on the principle of photoelectric coupling, the photo-coupler outputs a corresponding output signal after receiving the disable signal, so that the MOSFET is turned off. At this time, the closed loop formed is disconnected, and the heating member stops heating.

Therefore, the constant power output of the heating member is achieved by controlling the on duration and the off duration of the MOSFET, that is, controlling the duty ratio of the MOSFET.

Moreover, it should be noted that the disable signal output by the power calculation chip is a slow shutdown process for the MOSFET, which can effectively reduce, compared with a fast process, the EMI of the circuit and avoid the interference signal generated by the fast shutdown from affecting other devices in the circuit.

In summary, the heating control circuit of the embodiment of the present disclosure has the following advantages: 1) the circuit structure is simple and easy to use; 2) the heating control circuit can be connected to an AC power supply with various AC voltages to meet the needs of a wide voltage and is suitable for different AC power supply applications; 3) it is not necessary to reduce the voltage, thereby saving the cost; 4) the constant power output of the heating member is achieved, and the use security of the heating member is improved.

An embodiment of the present disclosure further provides a ventilator, including: a heating member; and the heating control circuit described in the above embodiments. The heating control circuit controls the output power of the heating member, so that the heating member can perform the heating work at a constant power. Preferably, the heating member is a heating plate.

The inventors have found that domestic ventilation treatment equipment and oversea ventilation treatment equipment adopt heating plates of different specifications currently. Taking a heating plate with a stable output power of 200W as an example, in countries where the civil AC standard voltage is 110V, the resistance value of the heating plate is 60.5 Q (ohms), and in China and other countries where the civil AC standard voltage is 220V, the resistance value of the heating plate is 242 Q .

For the above reason, when producing and assembling ventilation treatment equipment, it is necessary to confirm in advance the countries and regions where the ventilation treatment equipment will be sold to ensure that the heating plate and supporting software match other hardware.

The inventors further studied and found that there are two main reasons for adopting the above solution:
1) Confusing heating plates with different specifications of resistance values will lead to great risks. For example, if a 110V heating plate with a resistance value of 60.5 Q is used at 220V, the heating power of the heating plate will become 4 times that of normal conditions. It is easy to cause abnormal temperature rise due to excessive power, affecting the heating control and related fault judgment.
2) If a 220V heating plate with a resistance value of 242 Q is used at a voltage of 110V, the heating power of the heating plate will be only 1/4 of the normal rated power, which is seriously insufficient to meet the heating and humidification requirements of ventilation treatment equipment.

Since the consequences caused due to the first reason are very serious, the specifications of ventilation treatment equipment need to be distinguished when the ventilation treatment equipment is registered in different countries, which increases the difficulty of registration. However, since the civilian AC standard voltage is not only different between China and foreign countries, but also different in foreign countries, it is impossible to rule out the possibility that ventilation treatment equipment will be used in countries with different voltages. Even if it is registered separately, there is still a risk.

In addition, in some countries or regions where the quality of the power grid is unstable, the voltage of the operating power supply may fluctuate, which will cause the output power of the heating plate to fluctuate, which will also affect the therapeutic effect of the ventilation treatment equipment.

In view of the above problems, the inventor creatively proposed a method for maintaining power stability of the present disclosure, and the method proposed in the present disclosure is described in detail below.

Reference is made to FIG. 5 which is a flow chart of a method for maintaining power stability according to an embodiment of the present disclosure. The method includes steps described below.

At step S101, a target power value and an actual maximum power value are determined, and the target power value represents a power value required to be stably output by the ventilation treatment equipment.

When the ventilation treatment equipment is powered on and ready to start operating, the target power value and the actual maximum power value are determined first. The so-called target power value represents a power value required to be stably output by the heating plate of the ventilation treatment equipment. For example, if the heating plate needs to output a stable power of 100W, then the target power value is 100W.

The actual maximum power value refers to the maximum power value that the heating plate of the ventilation treatment equipment can achieve in actual operation. When the resistance value of the heating plate is determined, the actual maximum power value changes according to the working power supply value. Therefore, when the ventilation treatment equipment is actually operating, the method for determining the actual maximum power value specifically includes:
at step S1, acquiring an operating power supply value for a ventilation treatment equipment; and
at step S2, determining the actual maximum power value according to the working power supply value and the resistance value of the heating plate.

First, the working power supply value for the ventilation treatment equipment is obtained, which is the standard value of civil alternating current in the country or region where the ventilation treatment equipment is used. Then, the actual maximum power value is determined based on the working power supply value and the resistance value of the heating plate. The actual maximum power value can be calculated as follows:
actual maximum power value = (working power supply value)² / resistance value of the heating plate.

In the method for maintaining power stability proposed by the present disclosure, in order to achieve the object, the resistance value of the heating plate is determined based on the following criteria:
considering the standard voltage range of international civil electricity, the ventilation treatment equipment should be able to continuously output the target power value while operating, and the actual maximum power value should be not less than the target power value. For example, the standard voltage range of international civil electricity is 100~240V. If the target power value is 200W, the resistance value of the heating plate may be any value greater than 0 Q but not greater than 50 Q. 50 Q is obtained based on the following criteria: if the resistance value of the heating plate is selected to be greater than 50 Q, assuming it is 50.5 Q, then when the working power supply value for the ventilation treatment equipment is 100V, the actual maximum power value is 100²/50.5=198W, which is less than the target power value of 200W. Of course, if the resistance value of the heating plate is too small, the actual maximum power value will be much greater than the target power value under the same working power supply value. As a result, the current flowing through the heating plate will be too large, which may shorten the service life of the heating plate. Taking all the above factors into consideration, an optimal resistance value is 50 Q .

At step S 102, an overall control signal is output based on the target power value and the actual maximum power value. The overall control signal includes an enable signal and a disable signal. Specifically, for each output of the enable signal, the heating plate of the ventilation treatment equipment operates continuously for a preset duration; for each output of the disable signal, the heating plate stops operating for a preset duration.

After determining the target power value and the actual maximum power value, the overall control signal can be output according to the target power value and the actual maximum power value. The overall control signal includes an enable signal and a disable signal. When a heating control board receives the enable signal, the heating plate can be controlled to continuously operating for a predetermined duration.

Generally, a main control board in the ventilation treatment equipment generates the overall control signal, and the overall control signal includes the enable signal and the disable signal. The so-called enable signal is a signal that causes the heating plate to start operating; the so-called disable signal is a signal that causes the heating plate to stop operating.

The specific methods for outputting the overall control signal of the present disclosure may include the followings:
(1) outputting the overall control signal according to the duty ratio; and
(2) outputting the overall control signal by using an accumulation method.

Method (1) for outputting the overall control signal based on the duty ratio specifically includes steps below.

At step T1, the duty ratio is determined according to the target power value and the actual maximum power value.

For the sake of simplicity in calculation, a ratio of the target power value to the actual maximum power value can be directly used as the duty ratio, that is, duty ratio = target power value / actual maximum power value.

At step T2, the overall control signal is output according to the duty ratio.

After the duty ratio is obtained, the number of times that the overall control signal is output can be determined according to the duty ratio, and the number of times that the overall control signal is output is the value of the denominator in the duty ratio. As mentioned above, the overall control signal includes the enable signal and the disable signal. Once the disable signal is output, the heating plate stops operating for a predetermined duration. Then, according to the duty ratio and the number of times that the overall control signal is output, the number of times that the enable signal is output and the number of times that the disable signal is output are determined. The number of times that the enable signal is output is the value of the numerator in the duty ratio. Finally, the enable signal is output according to the number of times that the enable signal is output, and the disable signal is output according to the number of times that the disable signal is output.

If the enable signal is to be output multiple times, then the enable signal is output continuously multiple times after all the disable signals have been output. If the enable signal is to be output only once, then the enable signal is output once after all the disable signals have been output.

To explain the above method more clearly, a specific example is given. Assuming that the resistance value of the heating plate is 50 Q, the target power value is 100W, and the working power supply value is 200V, then the actual maximum power value is 2002/50=800W, and the duty ratio is 100/800=1/8. That is, the overall control signal is output 8 times, the enable signal is output once, and the disable signal is output 7 times. If the main control board operates with a cycle of 0.1 seconds, that is, sending a signal to the heating control board every 0.1 second, then a disable signal is sent to the heating control board within a period from 0 to 0.7 seconds, and the heating control board will control the heating plate to stop operating for 0.7 seconds after receiving the disable signal. At 0.8 seconds, an enable signal is sent to the heating control board, and the heating control board will control the heating plate to continuously work for 0.1 seconds after receiving the enable signal.

Next, from 0.9 to 1.5 seconds, the main control board sends a disable signal to the heating control board, after receiving the disable signal, the heating board controls the heating plate to stop operating for 0.7 seconds. The main control board sends an enable signal to the heating control board at 1.6 seconds, after receiving the enable signal, the heating board controls the heating plate to continuously work for 0.1 seconds. The above process is repeated until the ventilation treatment equipment stops operating, and during the above process, the power of the heating plate is always kept stable at 100W.

It can be understood that if the duty ratio is 2/7, the over control signal is output 7 times, among them, the enable signal is output 2 times, and the disable signal is output 5 times. If the main control board operates with a cycle of 0.1 second, that is, sending a signal to the heating control board every 0.1 second, then a disable signal is sent to the heating control board within a period from 0 to 0.5 seconds, and after receiving the disable signal, the heating control board will control the heating plate to stop operating for 0.5 seconds. At 0.6 seconds and 0.7 seconds, an enable signal is sent to the heating control board, after receiving the enable signal, the heating control board controls the heating plate to work continuously for 0.2 seconds. By proceeding in this manner, the power of the heating plate is always stably maintained at the target power value. It is understandable that when the working power supply value fluctuates, the actual maximum power value also changes. Accordingly, the duty ratio changes, the output number of the enable signal changes, but the power of the heating plate remains stable at the target power value.

The above method (2) for outputting the overall control signal by using the accumulation method specifically includes steps below.

At step V1, the target power value is taken as an incremental value.

At step V2, an overall control signal is output according to the incremental value and the actual maximum power value.

Instead of obtaining the duty ratio, this method directly uses the target power value as the incremental value, and outputs the overall control signal based on the incremental value and the actual maximum power value. An optimal manner is as follows:

Starting from 0, an incremental value is accumulated once in each preset cycle, and the accumulated value obtained from accumulating the incremental value once is compared with the actual maximum power value in each preset cycle. That is, in the first preset cycle, 0 is added to the target power value once to obtain an accumulated value, the magnitude of which is equal to the target power value, and after the accumulation, the accumulated value is compared with the actual maximum power value. In the second preset cycle, the accumulated value of the first preset cycle is used and added to the incremental value again, that is, in the second preset cycle, the accumulated value is equal to twice the target power value, and the accumulated value (twice the target power value) is compared with the actual maximum power value. In the third preset cycle, the accumulated value of the second preset cycle is used and added to the incremental value again, that is, in the third preset cycle, the accumulated value is equal to three times the target power value, and the accumulated value (three times the target power value) is compared with the actual maximum power value, and so on.

If the accumulated value in any preset cycle is less than the actual maximum power value, a disable signal is output in the preset cycle, and the heating plate stops operating for a predetermined duration each time the disable signal is output. If the accumulated value in any preset cycle is not less than the actual maximum power value, an enable signal is output in the preset cycle. Assuming that in the third preset cycle, the accumulated value (three times the target power value) is less than the actual maximum power value, then the disable signal is output in all of the first three preset cycles. Assuming that in the third preset cycle, the accumulated value (three times the target power value) is not less than the actual maximum power value, then a disable signal is output in the first two preset cycles, and an enable signal is output in the third preset cycle.

In a preset cycle following the cycle during which the enable signal is output, steps are repeated based on the difference: the incremental value is accumulated once in each preset cycle, and the accumulated value obtained through accumulating the incremental value once is compared with the actual maximum power value in each preset cycle. The so-called difference value is the difference value between the accumulated value in the preset cycle during which the enable signal is output and the actual maximum power value. Assuming that in the third preset cycle, the accumulated value (three times the target power value) is 130W which is not less than the actual maximum power value 120W, then a disable signal is output in both of the first two preset cycles, and an enable signal is output in the third preset cycle. In the fourth cycle, steps are repeated based on the difference of 10W (130-120=10W) as follows: in the fourth preset cycle, the incremental value is accumulated once, that is, 10 W is added to the target power value to obtain an accumulated value, and the accumulated value is compared with the actual maximum power value in the fourth preset cycle. The above steps are repeated until the ventilation treatment equipment stops operating.

The above specific example is used, assuming that the resistance value of the heating plate is 50 Q, the target power value is 100W, and the working power supply value is 200V. Then, the actual maximum power value is 200²/50=800W, the target power value of 100W is used as the increment value and the preset cycle is 0.1 second. Starting from 0 second, the target power value of 100W is accumulated every 0.1 seconds, so the accumulated value is 100W in 0.1 second cycle, which is less than the actual maximum power value of 800W. Thus, the main control board sends a disable signal to the heating control board in a 0.1 second cycle, after receiving the disable signal, the heating control board controls the heating plate to stop operating for 0.1 seconds. In the 0.2 second cycle, the accumulated value is 200W, which is less than the actual maximum power value of 800W, then the main control board sends a disable signal to the heating control board in a 0.2 second cycle. After receiving the disable signal, the heating control board controls the heating plate to stop operating for 0.1 second. In this way, until the accumulated value is 800W in the 0.8 second cycle, which is equal to the actual maximum power value of 800W, the main control board sends an enable signal to the heating control board in the 0.8 second cycle. After receiving the enable signal, the heating control board controls the heating plate to work continuously for 0.1 seconds. In the 0.9 second cycle, the above process is repeated again based on the difference value of 0 until the ventilation treatment equipment stops operating. During the above process, the power of the heating plate is always kept stable at 100W.

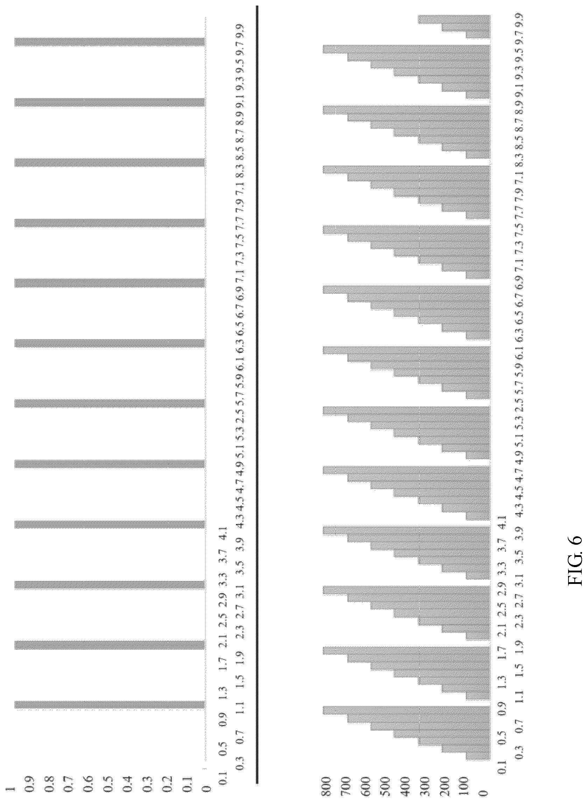

Reference is made to FIG. 6, which is a schematic diagram illustrating an enable signal output when the above method (2) for outputting an enable signal using the accumulation method is adopted. In FIG. 6, the upper half represents the output of the enable signal, with the horizontal axis representing time in seconds and the vertical axis representing the switch quantity, where 1 indicates outputting an enable signal. The lower half of FIG. 6 shows the accumulated value condition within each preset cycle, with the horizontal axis representing time in seconds and the vertical axis representing the incremental value. It can be visually understood that with a regular cycle of 0.8 seconds, from the beginning of the cycle to 0.7 seconds, the accumulated value increases from 100 to 700, and during this time, the disable signal is output. At the 0.8 seconds, the accumulated value is 800, the difference is 0, and the enable signal is output. It is understandable that when the working power supply value fluctuates, the actual maximum power value also changes accordingly. Thus, the preset cycle during which the accumulated value is not less than the actual maximum power value is different, and the cycle during which an enable signal is output also changes, but the power of the heating plate remains stable at the target power value.

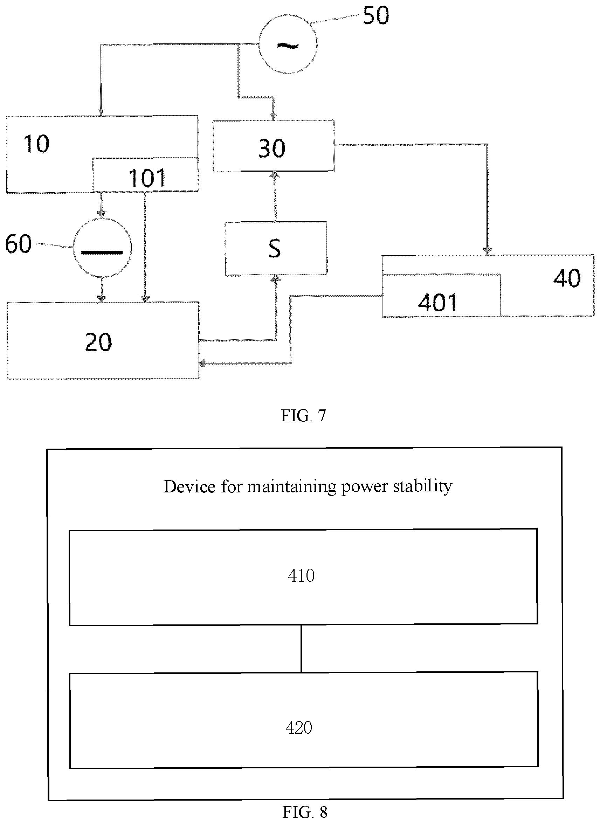

Based on the above method for maintaining power stability, a very simple change is made in the hardware of the ventilation treatment equipment in the present disclosure, that is, a voltage detection unit 101 is added to the operating power supply module 10 in the ventilation treatment equipment. Referring to FIG. 7, which is a schematic structural diagram of the ventilation treatment equipment, the ventilation treatment equipment includes an operating power supply module 10, a main control board 20, a heating control board 30, a heating plate 40, a temperature sensor 401, an AC power supply 50 (i.e., an operating power supply, using a civilian AC standard voltage) and a DC power supply 60. The main control board 20 outputs an overall control signal S to the heating control board 30, and receives the temperature value of the heating plate fed back by the temperature sensor 401.

During the actual operation of the ventilation treatment equipment, if the actual temperature of the heating plate 40 is too high, continued heating may pose certain risks, for example, the service life of the heating plate 40 is damaged due to overheating thereof, or the normal operation of other components may be interfered due to excessively high actual temperature of the heating plate 40. Therefore, the main control board 20 needs to continuously receive temperature signals from the temperature sensor 401, which represents the real-time temperature of the heating plate 40. When the real-time temperature is below a preset temperature, the main control board 20 performs the aforementioned step S102, i.e., outputting the overall control signal based on the target power value and the actual maximum power value. That is, a preset temperature is set, and the actual temperature of the heating plate 40 is not allowed to exceed this preset temperature during operation. When the actual temperature of the heating plate 40 is less than the preset temperature, the main control board 20 controls the operating state of the heating plate 40 according to step S102.

However, when the real-time temperature of the heating plate 40 is not less than the preset temperature, the main control board 20 continuously outputs a disable signal, causing the heating plate 40 to temporarily stop heating until the real-time temperature of the heating plate 40 decreases to below the preset temperature, then step S102 is executed again, i.e., the overall control signal is output based on the target power value and the actual maximum power value. That is to say, when the actual temperature of the heating plate 40 is not less than the preset temperature, the main control board 20 does not control the operating state of the heating plate 40 according to step S102, but controls the heating plate 40 to temporarily stop heating. The main control board 20 resumes controlling the operating state of the heating plate 40 according to the method of step S102 until the real-time temperature of the heating plate 40 decreases to below the preset temperature.

In the present disclosure, merely a voltage detection unit 101 is added to the operating power supply module 10 in an existing ventilation treatment equipment. This voltage detection unit 101 feeds back the detected voltage value of the AC power supply 50 to the main control board 20, so that the main control board 20 obtains the working power supply value of the ventilation treatment equipment, thereby achieving the method for maintaining power stability as described in steps S101 to S102.

In the above embodiment, according to the method for maintaining power stability of the present disclosure, it is not necessary to differentiated design and manufacture the resistance value of the heating plate of the ventilation treatment equipment. A single, unified resistance value can be used, without considering the standard voltage range of civil AC voltage. Two power values are used to control the output of the enable signal and the disable signal. Specifically, the heating plate continuously operates for a preset duration once the enable signal is output, and stops operating for a preset duration once the disable signal is output, so that the object of maintaining a stable output power of the heating plate is achieved. This achieves the object of stable power output from the heating plate

Since it is not necessary to design and manufacture heating plates with different resistance values, the cost of the ventilation treatment equipment is indirectly reduced. Moreover, even if the voltage of the operating power supply fluctuates, the output power of the heating plate is not affected. Based on the same heating plate resistance value, within the global standard voltage range of 100V to 240V, and even when the voltage of the operating power supply fluctuates, the object of maintaining a stable output power of the heating plate is achieved. This method has a high practical value.

It should be noted that the ventilation treatment equipment referred to in the present disclosure includes a ventilator and other equipment such as a high-flow humidified oxygen therapy device.

Based on the above method for maintaining power stability, the present disclosure further provides a device for maintaining power stability. Referring to FIG. 8, a block diagram of a device for maintaining power stability according to an embodiment of the present disclosure is shown.

The device includes: a determination module 410, configured to determine a target power value and an actual maximum power value, the target power value representing a power value required to be stably output by the ventilation treatment equipment; and an output module 420, configured to output an overall control signal based on the target power value and the actual maximum power value. The overall control signal includes an enable signal and a disable signal. Each time the enable signal is output, the heating plate of the ventilation treatment equipment continuously operates for a preset duration, and each time the disable signal is output, the heating plate stops operating for a preset duration.

Optionally, the output module 420 includes: a duty ratio determination sub-unit, configured to determine the duty ratio based on the target power value and the actual maximum power value; and a first overall control signal output sub-unit, configured to output the overall control signal according to the duty ratio.

Optionally, the first overall control signal output sub-unit is specifically configured to: determine, based on the duty ratio, the number of times that the overall control signal is output; determine, based on the duty ratio and the number of times that the overall control signal is output, the number of times that the enable signal is output and the number of times that the disable signal is output; output the enable signal according to the number of times that the enable signal is output, and output the disable signal according to the number of times that the disable signal is output.

Optionally, the output module 420 includes: an increment sub-unit, configured to take the target power value as an increment value; and a second overall control signal output sub-unit, configured to output the overall control signal based on the increment value and the actual maximum power value.

Optionally, the second overall control signal output sub-unit is specifically configured to: accumulate the increment value in each preset cycle starting from 0, and compare the accumulated value obtained through accumulating the increment value with the actual maximum power value in each preset cycle; if the accumulated value of any preset cycle is less than the actual maximum power value, then a disable signal is output in preset cycle, wherein the heating plate stops operating for a preset duration each time the disable signal is output; if the accumulated value in any preset cycle is not less than the actual maximum power value, then an enable signal is output in the preset cycle; in a preset cycle following the cycle during which the enable signal is output, the following steps are performed based on a difference value: accumulating the increment value once in each preset cycle, and comparing the accumulated value obtained by accumulating the increment value once with the actual maximum power value in each preset cycle, wherein the difference value is a difference value of the actual maximum power value and the accumulated value in the preset cycle during which the enable signal is output.

Optionally, the determination module 410 includes: an acquisition sub-unit, configured to acquire the working power supply value of the ventilation treatment equipment; and a determination sub-unit, configured to determine the actual maximum power value based on the working power supply value and the resistance value of the heating plate.

Optionally, the device also includes: a temperature reception module, configured to receive a temperature signal from a temperature sensor, the temperature signal representing the real-time temperature of the heating plate; a first execution module, configured to execute the steps of outputting the overall control signal based on the target power value and the actual maximum power value when the real-time temperature is less than the preset temperature; and a second execution module, configured to continuously output the disable signal when the real-time temperature is not less than the preset temperature, and execute the step of outputting the overall control signal based on the target power value and the actual maximum power value until the real-time temperature drops below the preset temperature.

Based on the above method for maintaining a stable power, the present disclosure also provides a ventilation treatment equipment including a main control board that is configured to perform any of the methods for maintaining stable power as described in steps S101 to S102. The ventilation treatment equipment can be a ventilator or a high-flow humidified oxygen therapy device.

It should be noted, the terms "comprising", "including" or any other variation thereof are intended to cover a non-exclusive inclusion, such that a process, method, article, or apparatus including a list of elements includes not only those elements but also other elements not expressly listed, or elements inherent to the process, method, article or apparatus. Without further limitations, an element defined by the phrase "including a ..." does not exclude the presence of additional identical elements in the process, method, article or apparatus including said element.

The above are merely embodiments of the present disclosure and are not intended to limit the present disclosure. For those skilled in the art, the present disclosure may have various modifications and variations. Any modification, equivalent substitution, improvement, etc. made within the spirit and principle of the present disclosure shall be included in the scope of the claims of the present disclosure.

## Claims

1. A heating control circuit, comprising a control device and a switching device, an input end of the control device being connected to an alternating current (AC) power supply, an output end of the control device being connected to a control end of the switching device, and an output end of the switching device being connected to a heating member;
wherein the control device is configured to generate, according to different output voltages of the AC power supply, a driving signal for controlling a duty ratio of the switching device to adaptively change, so that a power output from the switching device to the heating member is maintained constant.

2. The heating control circuit according to claim 1, wherein the switching device is a metal oxide semiconductor field effect transistor (MOSFET);
a gate of the MOSFET is connected to the control device, a source of the MOSFET is connected to a power ground terminal, and a drain of the MOSFET is connected to the input end of the heating member, wherein the gate of the MOSFET is configured as the control end.

3. The heating control circuit according to claim 1 or 2, further comprising a voltage divider circuit connected in parallel with a series circuit formed by the heating member and the switching device, and the voltage divider circuit comprises a first resistor and a second resistor connected in series.

4. The heating control circuit according to any one of claims 1 to 3, further comprising an isolation device disposed between the output end of the control device and the control end of the switching device.

5. The heating control circuit according to claim 4, further comprising a third resistor disposed between the isolation device and the switching device.

6. The heating control circuit according to any one of claims 1 to 5, further comprising a rectifying device, wherein the rectifying device is connected between the AC power supply and the heating member, and configured to convert an output voltage signal of the AC power supply into a DC voltage and provide the DC voltage to the heating member.

7. The heating control circuit according to any one of claims 1 to 6, wherein the heating control circuit further comprises a voltage acquisition device, wherein the voltage acquisition device is connected between the AC power supply and the control device, and is configured to sample the output voltage signal of the AC power supply and provide the output voltage signal of the AC power supply to the control device.

8. The heating control circuit according to claim 7, wherein the voltage acquisition device is a mutual inductor,
an input end of the mutual inductor is connected to the AC power supply, and an output end of the mutual inductor is connected to the input end of the control device.

9. The heating control circuit according to any one of claims 1 to 8, wherein the control device is a power calculation chip.

10. A ventilator, comprising:
a heating member; and
the heating control circuit according to any one of claims 1 to 9.
